Europäisches Patentamt

European Patent Office

Office européen des brevets

(11) Publication number: **0 356 594**
**A1**

## EUROPEAN PATENT APPLICATION

(21) Application number: 88402059.5

(22) Date of filing: 08.08.88

(51) Int. Cl.5: **A61N 2/08** , **A47G 9/02**

(43) Date of publication of application:
**07.03.90 Bulletin 90/10**

(84) Designated Contracting States:
**AT BE CH DE ES FR GB GR IT LI LU NL SE**

(71) Applicant: **J.L.S. CORP.**
**No 1-1, 3-chome, Higashiikebukuro**
**Toshima-ku Tokyo(JP)**

(72) Inventor: **Yamaguchi, Tomoyoshi**
**c/o J.L.S.Corp. No. 1-1, 3-chome**
**Higashiikebukuro**
**Toshima-ku Tokyo(JP)**

(74) Representative: **Rodhain, Claude et al**
**Cabinet Claude Rodhain 30, rue la Boétie**
**F-75008 Paris(FR)**

(54) Feather bed quilts and method for fixing permanent magnets to cover of bed clothing.

(57) Feather bed quilts, comprising a thick feather bed quilt (1) and a thin feather bed quilt (2), and wherein the thick bed quilt (1) is detachably attached in an entire circumference of the rear side, and the thin feather bed quilt (2) is the same in an entire circumference of one side, and at least the thin feather bed quilt is arranged with a plurality of permanent magnets (6) in an inside thereof. Due to the permanent magnets (6) arranged to the side of contacting the human body, the blood circulation is accelerated during sleeping.

A method for fixing a plurality of permanent magnets (6) to an inner cover (17) of bedclothing is disclosed.

# FIG. 2

EP 0 356 594 A1

# FEATHER BED QUILTS AND METHOD FOR FIXING PERMANENT MAGNETS TO COVER OF BEDCLOTHING

## BACKGROUND OF THE INVENTION

### Field of the Invention :

The present invention relates to feather bed quilts and a method for fixing a plurality of permanent magnets to an inner side of the cover of the above.

### Description of the Prior Art :

Naturally, a thin quilt is used in the hot season, and a thick quilt is used in the cold season. Therefore, seasonal quilts are respectively required.

However, it is an economical burden to get ready for thin and thick ones of several sheets such as expensive feather quilts, and to uselessly allow the summer quilt to rest in the winter, and vise versa. In addition, the feather quilt should always absorb the air, otherwise physiological functions thereof as shrinkage, moisture absorption, water repulsion and others, become weak, and warming effect could be considerably diminished. So, non-use for a long period of time is not preferable.

When the summer quilts are used by merely putting one on the other in the winter, light ones such as the feather quilts easily slide off. For sound an sweet sleep, the blood circulation be smooth all over the body to remove fatigue and stiffness of the shoulders. However, although the feather quilt has the warming effect, it does not accelerate the blood circulation.

## SUMMARY OF THE INVENTION

As said, the foregoing feather quilts could not be distinguished seasonably, and has not been widely distributed due to high price and does not have such a function to accelerate the blood circulation to relieve the fatigue.

The main object of the invention is to provide feather quilts which could be used seasonally and economically with the blood circulation.

It is another object of the present invention to provide a method for quickly and surely fixing a permanent magnet to a cover of bedclothing in a simple manner.

It is still another object of the present invention to provide a method for fixing the permanent magnet to the cover of bedclothing without any fear that some pieces of harmful rivet joints remain in the bedclothing, so as to achieve 100% safety.

Other and further objects, features and advantages of the present invetion will appear more fully from the following description.

## BRIEF DESCRIPTION OF THE DRAWINGS

Fig. 1 shows a perspective view of an embodiment of the invention ;

Fig. 2 is a view showing a combination of an upper and lower bed quilts ;

Fig. 3 is a view showing arrangement of permanent magnets ; and

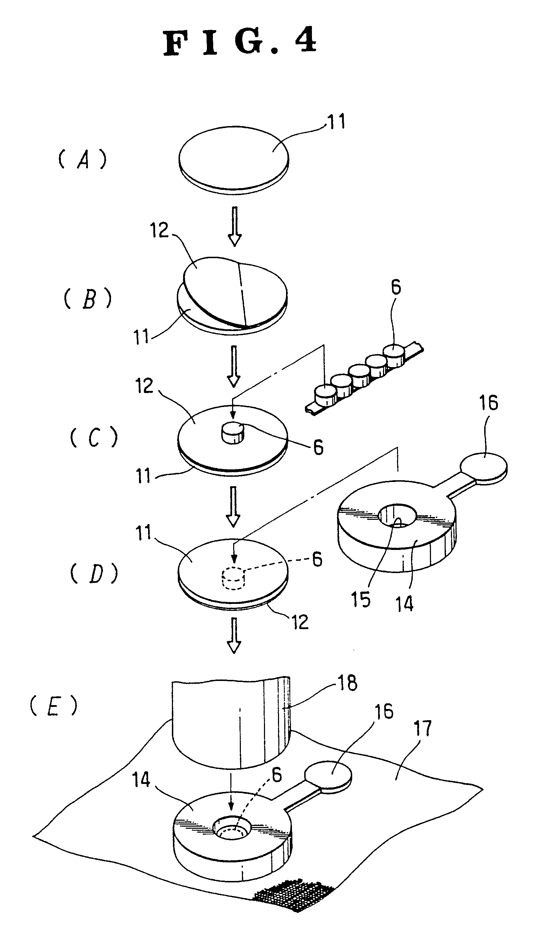

Fig. 4 is a view for illustrating sequential steps of the method of the present invention.

## DESCRIPTION OF THE PREFERRED EMBODIMENT

The most preferable embodiment of the present invention will be described in detail with reference to the accompanying drawings.

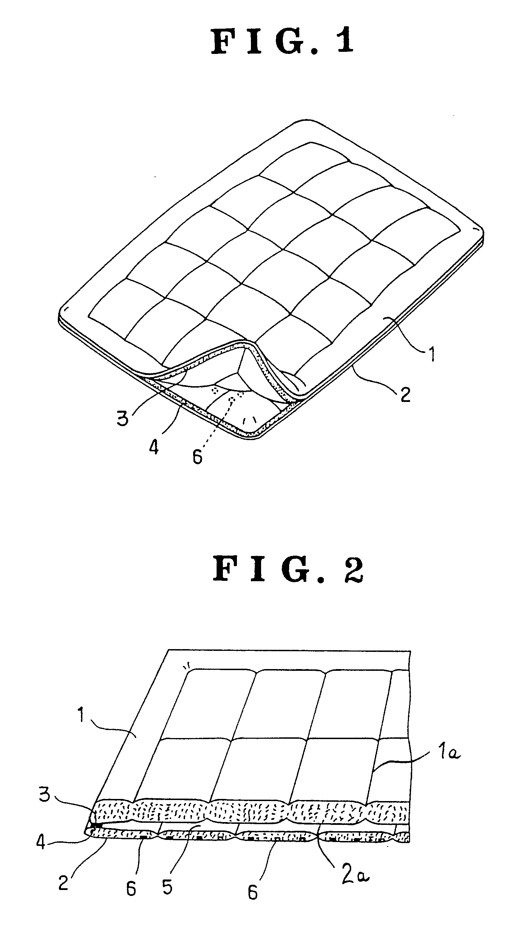

The drawings show preferable embodiments of the invention, in which the reference numeral 1 designates a thick feather quilt and 2 shows a thin feather quilt. At least the feather quilt 1 is sewn with a surface cloth and a lining cloth. The quilts 1 and 2 are processed to avoid biasing of feathers and cottons by quiltings, and they may be used alone. The quilts 1, 2 may be used together, and stitches 1a, 2a should not be met (Fig. 2) at use. Thereby, so-called cold points are not made, and the warming effect could be secured. For combining the two quilts 1 and 2, holding means are provided such as velvet fasteners 3 or snaps on the entire circumferential edge of the lining velvet fasteners 4, so as to make one set of the quilts 1, 2. The velvet fastener is desirable since it is easily attached and detached, and can perfectly seal the allover circumference. If occasion demands, a further thin quilt is held as a third layer between the quilts 1 and 2 by holding means on the surface and lining sides. These quilts are combined closely with a new air layer 5, and appear as a thick one.

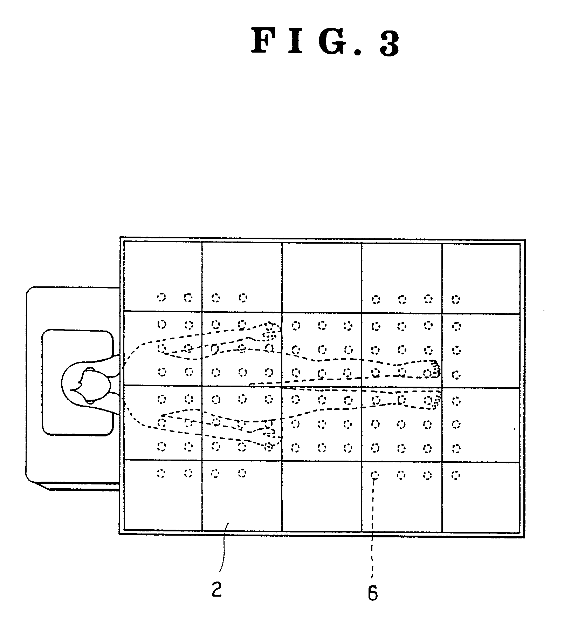

In the drawings, the numeral 6 designated permanent magnets of 2 poles in one side to be arranged (e.g., 80 to 90) in an inner side of the quilt contacting a human body. Fig. 3 shows the arrangement of the permanent magnets 6.

The method of the present invention is provided with sequential steps having the following order.

Step (A) providing a patch 11 :

The same fabric as that of a cover 17 of bedclothing is cut into a suitable shape such as a circular shape and the like, provided that cutting of such fabric is generally conducted in the following step (B) ;

Step (B) for applying a bonding-agent sheet 12 to the patch 11 :

A plurality of sheets from each of which the patch 11 is constructed and a plurality of the bonding-agent sheets 12 are alternately stacked into a pile which is punched to form a suitable form such as a circulat form and the like so as to produce a plurality of the patches 11 and the bonding-agent sheets 12 at once. The patch 11 and the bonding-agent sheet 12 are heated to a temperature of up to a complete-fusion temperature of the bonding-agent sheet 12 so as to be temporarily welded to each other. In this case, the bonding-agent sheet 12 is made of a material having its complete-fusion temperature of about 150°C and assumes a wafer-like form ;

Step (C) for temporarily fixing a permanent magnet 6 to a center of the bonding-agent sheet 12 ;

The permanent magnet 6 is slightly heated, and then positioned in the bonding-agent sheet 12 so that the permanent magnet 6 is temporarily fixed to the bonding-agent sheet 12 under the effect of a temperature of the thus heated permanent magnet 6, whereby an assembly 6, 11, 12 consisting of the permanent magnet 6 and the bonding-agent sheet 12 is prepared ;

Step (D) for placing the assembly 6, 11, 12 on the cover 17 of bedclothing in a condition in which the assembly 6, 11, 12 abuts on the cover 17 at a side of the permanent magnet 6 of the assembly 6, 11, 12 through the bonding-agent sheet 12 of which assembly a platen 14 is placed on the cover 17 :

The assembly 6, 11, 12 consisting of the permanent magnet 6 and the bonding-agent sheet 12 is placed on the cover 17 of bedclothing in a condition in which the assembly 6, 11, 12 abuts on the cover 17 at a side of the permanent magnet 6 of the assembly 6, 11, 12 so that a surface of the permanent magnet 6 is brought into a direct contact with the cover 17 of bedclothing. Then, the platen 14 is made of a material such as copper and the like having a large thermal conductivity, while shaped into a form similar to that of the patch 11, provided that the platen 14 has a central hole 15 which is larger in diameter than the permanent magnet 6. The central hole 15 of the platen 14 may be constructed of a through-hole as shown in the drawing or a mere concave portion. In addition, the platen 14 may be provided with a grip portion 16 as required. When the platen 14 placed on the patch 11 of the assembly 6, 11, 12 is slightly depressed, the permanent magnet 6 of the assembly 6, 11, 12 is received in the central hole 15 of the platen 14, while the patch 11 around the permanent magnet 6 is brought into a close contact with the cover 17 of bedclothing through the bonding-agent sheet 12 ; and

Step (E) for heating the platen 14 ;

In case that a suitable heating means such as a heater and the like is provided in the platen 14, the platen 14 is heated by energizing such heating means. On the other hand, in case that the platen 14 is not provided with any heating means as shown in the drawing, an independent heating instrument 18 is brought into contact with the platen 14 to heat the same so that the bonding-agent sheet 12 of the assembly 6, 11, 12 is heated to its melting point, whereby the bonding-agent sheet 12 of the assembly 6, 11, 12 is completely melted to cause the patch 11 of the assembly 6, 11, 12 to be completely welded to the cover 17 of bedclothing in a condition in which the permanent magnet 6 of the assembly 6, 11, 12 is encapsulated in the patch 11 and the cover 17.

In a manner as described above, a plurality of the permanent magnets 6 are fixed to an inner-side cover 17 of bedclothing, which inner cover 17 abuts on a body of the user. As a result, the permanent magnets 6 is brought into contact with the body of the user through the cover 17 of bedclothing to encourage his blood flow during sleeping under the influence of magnetism.

## Claims

1 - Feather bed quilts, comprising a thick feather bed quilt (1) and a thin feather bed quilt (2), and wherein the thick bed quilt (1) is detachably attached in an entire circumference of a rear side, and the thin feather bed quilt (2) is the same in an entire circumference of one side, and at least the thin feather bed quilt is arranged with a plurality of permanent magnets (6) in a inside thereof.

2 - Feather bed quilts as claimed in claim 1, wherein when the thick (1) and the thin(2) feather bed quilts are combined, stitched formations are not met each other.

3 - A method for fixing a permanent magnet (6) to a cover of bed-clothing, comprising the steps of :

providing a patch (11) assuming a suitable shape such as a circular shape and the like ;
applying a bonding-agent sheet (12) to said patch ;

fixing temporarily a permanent magnet (6) to a center of said bonding-agent sheet to prepare an assembly consisting of said patch (11), said bonding-agent sheet (12) and said permanent magnet (6) ;

placing said assembly on a cover (17) of bedclothing in a condition in which said permanent magnet of said assembly abuts on said cover of bedclothing ;

heating said platen (14) to melt said bonding-agent sheet (12) of said assembly so as to bond said patch (11) of said assembly to said cover (17) of bedcloting.

4 - The method for fixing the permanent magnet to the cover of bedclothing as set forth in claim 3, wherein a heating means (18) is incorporated in said platen.

# FIG. 1

# FIG. 2

# F I G . 3

2    6

# FIG. 4

(A)

(B)

(C)

(D)

(E)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | FR-A-1 254 960  (RAULT)<br>* abstract; figure 4 *<br>--- | 1 | A 61 N    2/08<br>A 47 G    9/02 |
| Y,A | FR-A-2 458 257  (ETABLISSEMENTS A. LEOPOLD & FILS AMBOISE)<br>* claims 1-5; figure 1 *<br>--- | 1,2 | |
| A | CH-A-  343 086  (WESTERWINTER)<br>* page 3, line 22 - line 29 *<br>--- | 1, 2 | |
| A | GB-A-1 596 314  (FUJIMOTO COMPANY)<br>* figures 1-6 *<br>--- | 3 | |
| A | EP-A-0 122 293  (NIHON KENKOZOSHIN KENKYUKAI CO.)<br>----- | | |

TECHNICAL FIELDS
SEARCHED (Int. Cl.5)

A 61 N
A 47 G

The present search report has been drawn up for all claims

| Place of search | Date of completion of the search | Examiner |
|---|---|---|
| THE HAGUE | 30-03-1989 | BEUGELING G.L.H. |